# EUROPEAN PATENT APPLICATION

(11) **EP 4 056 181 A1**
(43) Date of publication of application: **14.09.2022**
(21) Application number: 20883709.6
(22) Date of filing: 06.11.2020
(51) Int. Cl.: A61K 31/505, A61K 31/506, C07D 239/48, C07D 401/12, A61P 37/06

(54) **USE OF COMPOUND IN PREVENTING OR TREATING GRAFT VERSUS HOST DISEASE**

(30) Priority: 08.11.2019 CN 201911097630
(71) Applicant: Shenzhen Chipscreen Biosciences Co., Ltd., Shenzhen, Guangdong 518057 (CN)
(72) Inventor: SHAN, Song, Shenzhen, Guangdong 518057 (CN); YU, Jindi, Shenzhen, Guangdong 518057 (CN); PAN, Desi, Shenzhen, Guangdong 518057 (CN); XIN, Lijun, Shenzhen, Guangdong 518057 (CN); WANG, Shigang, Shenzhen, Guangdong 518057 (CN); ZHANG, Yu, Shenzhen, Guangdong 518057 (CN)
(74) Representative: J A Kemp LLP
(86) International application number: PCT/CN2020/127064
(87) International publication number: WO 2021/088975

(57) **Abstract**

Use of a compound in preventing or treating a graft versus host disease (GVHD), especially use of the compound of formula (I) or a pharmaceutically acceptable salt thereof in preparing a drug for preventing or treating GVHD

## Description

### TECHNICAL FIELD

The present invention relates to a use of a compound in preparing a drug for preventing or treating a graft versus host disease (GVHD).

### BACKGROUND ART

GVHD is the most common complication after allogeneic hematopoietic stem cell transplantation (HSCT). Despite strong immunosuppressive prophylaxis, GVHD may occur even while donors are perfectly matched (identical human leukocyte antigen (HLA)) siblings. This is a result of an interaction between antigen-presenting cells of a receptor and mature cells of the donor. Immune cells in a graft may also cause the immune damage to host tissues, and cause a non-disease-recurrent death. GVHD occurs in 20-80% of patients who receive the transplantation and survive for a long-term, and is a common problem which needs to be concerned in a clinical transplantation surgery. According to data of the Hematology Branch of the Chinese Medical Association, more than 5,000 patients receive allogeneic bone marrow transplantation in China every year, and all of the patients need to receive GVHD prevention and treatment.

GVHD is usually divided into acute GVHD (aGVHD) and chronic GVHD (cGVHD) according to the occurrence time. aGVHD usually occurs within 3 months after transplantation, and occurs within 6 months at the latest. Three organs mainly involved in aGVHD are a skin, a gastrointestinal tract and a liver; and cGVHD may occur at any time periods after 6 months of the transplantation, and barrier tissues attacked by cGVHD include mucosal areas of an eye, a mouth, an intestine or a genital, and an epithelial barrier involving a mucosa in bronchiolitis obliterans. The median time of an immunosuppressive therapy in a transplant receptor is usually 2-3 years.

The first-line treatment of aGVHD is an intravenous injection of adrenal corticosteroid methylprednisolone, and the second-line treatment for a patient who fails to respond to a hormone therapy includes tacrolimus or mycophenolate mofetil or combination with methylprednisolone. A small-molecular targeted drug Janus kinase (JAK) inhibitor Ruxolitinib is recently approved for the second-line treatment in the acute phase but is not yet marketed in China. GVHD appears earlier, the prognosis is worse, so the active treatment is required clinically.

A standard first-line scheme for the treatment of cGVHD is to start the combined administration of prednisolone and azathioprine early, and gradually reduce the dose. If the patient is intolerant, the prednisolone combined with cyclosporine may also be used. For the second-line treatment, tacrolimus or rituximab or imatinib or pentostatin or the like may be used. While a second-line single drug is ineffective, the combination of the second-line drugs is preferred. There are fewer drugs to enter third-line treatment options, pulse-dose hormone therapy or mycophenolate mofetil or methotrexate or the like may be used.

Activation of the T lymphocyte is a primary link in inducing the occurrence and development of GVHD. Both chemoradiotherapy before the transplantation and GVHD after the transplantation may cause damage to a tubular epithelial cell (TEC), resulting in the limitation of a standard central tolerance mechanism and the inability to remove reactive T and B lymphocytes, especially a naive T lymphocyte (unexposed to an antigen) induces the more severe GVHD. The occurrence of aGVHD may involve the regulation of an inflammatory mediator and a T lymphocyte subset of GVHD by Janus kinase/signal transducer and activator of transcription (JAK/STAT) and natural kill cell-κB (NK-κB) pathways.

Drugs for the prevention or treatment of GVHD are still needed, particularly drugs for improving the survival rate or weight gain of the patient with GVHD

### SUMMARY OF THE INVENTION

A purpose of the present invention is to provide a drug for preventing or treating GVHD Another purpose of the present invention is to provide a drug for improving the survival rate or the weight gain of a patient with GVHD.

In one aspect, the present invention relates to a use of a compound of Formula (I) or a pharmaceutically acceptable salt thereof in preparing a drug for preventing or treating GVHD, 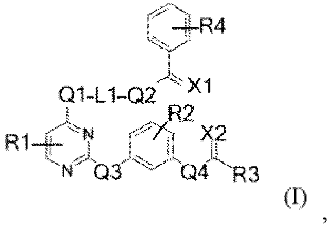 wherein,
R1 is halogen,
R2 is halogen,
R3 is selected from C₁₋₆ alkyl, C₂₋₆ alkenyl or C₂₋₆ alkynyl,
R4 is selected from -CN or halogen,
X1 is selected from O, S, NH or CH₂,
X2 is selected from O, S, NH or CH₂,
Q1 is selected from NH, CH₂, O or S,
Q2 is selected from NH, CH₂, O or S,
Q3 is selected from NH, CH₂, O or S,
Q4 is selected from N-R5 or CH-R5, wherein R5 is selected from C₁₋₆ alkyl, C₂₋₆ alkenyl or C₂₋₆ alkynyl, and
L1 is selected from C₁₋₆ alkylene, C₂₋₆ alkenylene or C₂₋₆ alkynylene.

In one aspect, in Formula (I), R3 is C₂₋₆ alkenyl, R4 is -CN, X1 is O, X2 is O, Q1 is NH, Q2 is NH, Q3 is NH, Q4 is N-R5, R5 is C₁₋₆ alkyl, and L1 is C₁₋₆ alkylene.

In one aspect, the compound of Formula (I) is a compound CS12192:

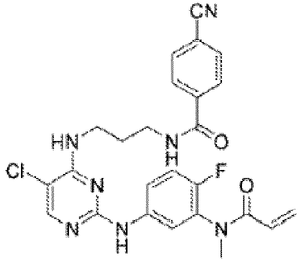

In one aspect, the GVHD is aGVHD or cGVHD

In one aspect, the drug is used to improve the survival rate of a patient with GVHD

In one aspect, the drug is used to improve the weight gain of a patient with GVHD

In one aspect, the drug further includes another active ingredient for preventing or treating GVHD. Preferably, the another active ingredient is selected from glucocorticoid, calcineurin inhibitor, mycophenolate mofetil, sirolimus, pentostatin, anti-CD25 monoclonal antibody, anti-TNFa monoclonal antibody, ciclosporin, methotrexate, thalidomide, or rapamycin. Preferably, the glucocorticoid is selected from prednisone or methylprednisolone.

In one aspect, the present invention relates to a method for preventing or treating GVHD, including administering a compound of Formula (I) to a subject in need thereof. Preferably, in Formula (I), R3 is C₂₋₆ alkenyl, R4 is -CN, X1 is O, X2 is O, Q1 is NH, Q2 is NH, Q3 is NH, Q4 is N-R5, R5 is C₁₋₆ alkyl, and L1 is C₁₋₆ alkylene. More preferably, the compound of Formula (I) is a compound CS12192. Preferably, the method may, for example, improve a survival rate of a patient with GVHD or improve a weight gain of the patient with GVHD. Preferably, the GVHD is aGVHD or cGVHD

In one aspect, the method further includes administering another active ingredient for preventing or treating GVHD to the subject. Preferably, the another active ingredient is selected from glucocorticoid, calcineurin inhibitor, mycophenolate mofetil, sirolimus, pentostatin, anti-CD25 monoclonal antibody, anti-TNFa monoclonal antibody, ciclosporin, methotrexate, thalidomide, or rapamycin. Preferably, the glucocorticoid is selected from prednisone or methylprednisolone.

The drug of the present invention may be used for preventing or treating GVHD, for example, the survival rate or the body weight of the patient with GVHD is improved.

### BRIEF DESCRIPTION OF THE DRAWINGS

Fig. 1 is a diagram showing an effect of compound CS12192 on mouse survival rate in a GVHD model; and
Fig 2 is a diagram showing an effect of compound CS12192 on mouse body weight in a GVHD model.

### DETAILED DESCRIPTION OF THE INVENTION

Unless otherwise defined, all technical and scientific terms used herein have the same meaning as commonly understood by those of ordinary skill in the art. In the case of conflicting, this document including definitions is used as a standard. Preferred methods and materials are described below, but methods and materials similar or equivalent to those described herein may be used for implementing or testing the present invention. The materials, methods, and examples disclosed herein are illustrative only, and not intended to limit.

The present invention provides a drug for preventing or treating GVHD. In one aspect, the present invention provides a drug for improving a survival rate or a weight gain of a patient with GVHD. In one aspect, the drug of the present invention has the better effects and/or fewer adverse reactions than drugs known in the field.

In one aspect, a compound of Formula (I) of the present invention, especially CS12192, may effectively treat GVHD, has a satisfactory therapeutic benefit rate for a GVHD model mouse, and has the drug effective activity superior to a first-line treatment drug adrenocortical hormone.

In one aspect, the present invention relates to a use of a compound of Formula (I) or a pharmaceutically acceptable salt thereof in preparing a drug for preventing or treating GVHD, 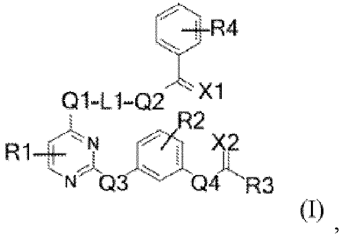 wherein,
R1 is halogen,
R2 is halogen,
R3 is selected from C₁₋₆ alkyl, C₂₋₆ alkenyl or C₂₋₆ alkynyl,
R4 is selected from -CN or halogen,
X1 is selected from O, S, NH or CH₂,
X2 is selected from O, S, NH or CH₂,
Q1 is selected from NH, CH₂, O or S,
Q2 is selected from NH, CH₂, O or S,
Q3 is selected from NH, CH₂, O or S,
Q4 is selected from N-R5 or CH-R5, wherein R5 is selected from C₁₋₆ alkyl, C₂₋₆ alkenyl or C₂₋₆ alkynyl, and
L1 is selected from C₁₋₆ alkylene, C₂₋₆ alkenylene or C₂₋₆ alkynylene.
In one aspect, the halogen is, for example, fluorine, chlorine, bromine or iodine.

In one aspect, the alkyl is, for example, C₁, C₂, C₃, C₄, C₅, or C₆ alkyl, such as methyl, ethyl, propyl, isopropyl, butyl, isobutyl, tert-butyl, pentyl, and hexyl.

In one aspect, the alkenyl is, for example, C₂, C₃, C₄, C₅, or C₆ alkenyl, such as vinyl, propenyl, butenyl, pentenyl, and hexenyl.

In one aspect, the alkynyl is, for example, C₂, C₃, C₄, C₅, or C₆ alkynyl, such as ethynyl, propynyl, butynyl, pentynyl, and hexynyl.

In one aspect, while a chemical bond of a substituent in Formula (I) is intersected with a chemical bond between two adjacent ring atoms of a cyclic moiety, it means that the substituent may be linked to any possible linkage positions of the cyclic moiety.

For example, in Formula (I),

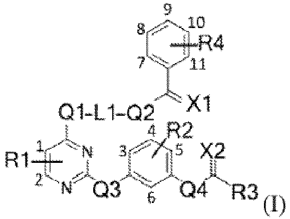

R1 may be linked with a position 1 or 2, preferably linked with the position 1; R2 may be linked with a position 3, 4, 5 or 6, preferably linked with the position 5; and R4 may be linked with a position 7, 8, 9, 10 or 11, preferably linked with the position 9.

In one aspect, in Formula (I), R3 is C₂₋₆ alkenyl, preferably a vinyl, R4 is -CN, X1 is O, X2 is O, Q1 is NH, Q2 is NH, Q3 is NH, and Q4 is N-R5, R5 is C₁₋₆ alkyl, preferably a methyl, and L1 is C₁₋₆ alkylene, preferably a propylidene.

In one aspect, the compound of Formula (I) is a compound CS12192:

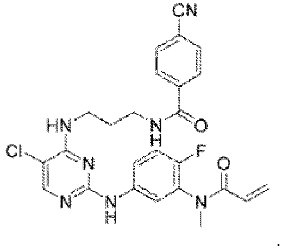

The chemical name of the compound CS12192 is N-(3-((5-chloro-2-((4-fluoro-3-(N-methacrylamido)phenyl)amino)pyrimidin-4-yl)amino) propyl)-4-nitrilebenzamide.

Embodiment 7 of Chinese patent CN105399685B discloses compound CS12192 and a preparation method thereof.

In one aspect, the GVHD is aGVHD or cGVHD.

In one aspect, the drug is used to improve survival rate of a patient with GVHD

In one aspect, the drug is used to improve weight gain of a patient with GVHD

In one aspect, the drug further includes another active ingredient for preventing or treating GVHD. Preferably, the another active ingredient is selected from glucocorticoid, calcineurin inhibitor, mycophenolate mofetil, sirolimus, pentostatin, anti-CD25 monoclonal antibody, anti-TNFa monoclonal antibody, ciclosporin, methotrexate, thalidomide, or rapamycin. Preferably, the glucocorticoid is selected from prednisone or methylprednisolone.

In one aspect, the present invention relates to a method for preventing or treating GVHD, including administering a compound of Formula (I) to a subject in need thereof.

In one aspect, the subject is a mammal, such as a human.

In one aspect, the drug of the present invention may be an injection, a tablet, a pill, a lozenge, a soft capsule, a hard capsule, a granule, a powder, a solution, a suspension, a syrup, and any other suitable dosage forms. In one aspect, the drug of the present invention may be administered orally. In one aspect, the drug of the present invention may be administered parenterally, for example, by intraperitoneal, intramuscular, intraarterial, intravenous, subcutaneous, intradermal and other routes.

In one aspect, in addition to the active ingredient, the drug of the present invention further includes a pharmaceutically acceptable adjuvant. As the pharmaceutically acceptable adjuvant, it may be listed as, for example, a lubricant, a binder, a filler, a preservative, a surfactant, a colorant, a flavoring agent, an emulsifier, a suspending agent, a diluent, a gelling agent, a disintegrant, a pH adjuster, and a solubilizer. Those skilled in the art know that these adjuvants may be appropriately selected according to the appropriate dosage form, and the content thereof may be changed according to specific needs.

### Embodiment

The following embodiments show the use of the compound of Formula (I), CS12192, in prevention and treatment of GVHD

The embodiments of the present invention disclose the use of compound of Formula (I), CS12192, in prevention and treatment of GVHD.

The present invention is further described in detail below in combination with the specific embodiments and with reference to data. It should be understood that these embodiments are intended to describe the present invention only, and not to limit a scope of the present invention in any way. It should be particularly pointed out that all similar replacements and modifications are apparent to those skilled in the art, and they are regarded to be included in the present invention. In the following embodiments, various processes and methods which are not described in detail are conventional methods well-known in the art.

### Embodiment 1. Pharmacodynamic evaluation of CS12192 on GVHD model induced by mouse allogeneic bone marrow transplantation

This study evaluates a pharmacodynamics effect of a test drug CS12192 in a GVHD model induced by mouse allogeneic bone marrow transplantation. In the GVHD model, 6-8 week old of two types of C57BL/6 (H-2b) and BALB/c (H-2d) male mice are used as donor and receptor animals respectively. Wherein, C57BL/6 mice are the donor mice, and BALB/c mice are the receptor mice.

The receptor mice are sent to an irradiation center to receive 8.5 Gry systemic irradiation on the day before transplantation, and after the irradiation, the mice are randomly divided into groups according to the body weights of the mice. On day 0, after 3-5% isoflurane pre-anesthesia, the donor mice are killed by cervical dislocation, and spleens are excised in a sterile environment, ground, and filtered by a sterile filter. Then red blood cells are digested with ammonium chloride buffer solution, lymphocytes are washed twice with RPMI 1640 culture solution, and finally resuspended in RPMI 1640 culture solution containing 10% fetal bovine serum, to prepare a lymphocyte suspension.

The receptor mice are divided into 6 groups, and each group includes 10 mice. Wherein the same number of BALB/c(H-2d) mouse cells are injected into tail veins of syngeneic control mice of the first group; groups 2-5 are allogeneic bone marrow transplantation, including a vehicle group, a positive drug group and CS12192 high and low two-dose administration groups, each BALB/c(H-2d) receptor mouse is injected with 0.5 ml of the RPMI 1640 culture solution by the tail vein, wherein 10×10⁶ bone marrow cells and 6.25×10⁶ splenocytes of allogeneic C57BL/6(H-2b) donor mice are contained; and an irradiation control group does not undergo any cell transplantation. The grouping and dosing situations of each group are shown in Table 1.

**Table 1. Dosing group and dosing regimen**

| Group | Test drug | Dose (mg/kg) | Dosing regimen |
|---|---|---|---|
| Syngeneic bone marrow transplantation | Vehicle | - | Twice a day |
| Allogeneic bone marrow transplantation | Vehicle | - | Twice a day |
| Positive drug | Prednisolone | 20 | Once a day |
| Low dose group | CS12192 | 40 | Twice a day |
| High dose group | CS12192 | 80 | Twice a day |
| Irradiation group | Vehicle | - | Twice a day |

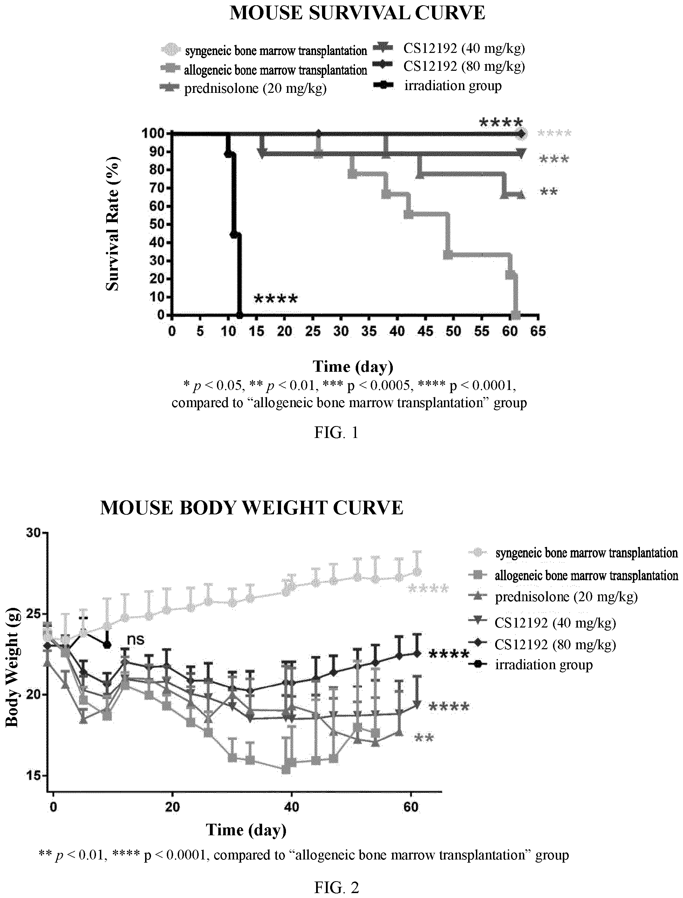

Experimental results of CS12192 against the mouse GVHD model (FIG. 1) show that the mice are reared for 61 days after allogeneic bone marrow transplantation, the survival rate of the allogeneic bone marrow transplantation control group is 0, and the survival rate of the positive drug prednisolone group is 67% (*p* < 0.01 relative to the vehicle control), and CS12192 is administered at two doses of 40 and 80 mg/kg twice a day, the survival rates at 61 days are 89% and 100% respectively (*p <* 0.0005 and *p* < 0.0001 relative to the vehicle control respectively); and at the same time, the weight gains of the mice of CS12192 two-dose groups are also significantly better than that of the positive drug prednisolone group, and it has a statistical increase advantage relative to the solvent control group (FIG. 2). The above experimental results show that CS12192 may significantly improve the survival rate of the GVHD model mice and obtain the comprehensive treatment benefits, it is indicated that GVHD is an effective indication for CS12192.

Although certain features of the present invention are already illustrated and described herein, many modifications, replacements, changes and equivalents may occur to those skilled in the art. Therefore, it should be understood that the appended claims are intended to cover all such modifications, replacements, changes and equivalents falling within the true spirit of the present invention.

## Claims

1. Use of a compound of Formula (I) or a pharmaceutically acceptable salt thereof in preparing a drug for preventing or treating a graft versus host disease (GVHD), 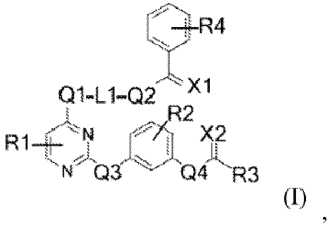 wherein,
R1 is halogen,
R2 is halogen,
R3 is selected from C₁₋₆ alkyl, C₂₋₆ alkenyl or C₂₋₆ alkynyl,
R4 is selected from -CN or halogen,
X1 is selected from O, S, NH or CH₂,
X2 is selected from O, S, NH or CH₂,
Q1 is selected from NH, CH₂, O or S,
Q2 is selected from NH, CH₂, O or S,
Q3 is selected from NH, CH₂, O or S,
Q4 is selected from N-R5 or CH-R5, wherein R5 is selected from C₁₋₆ alkyl, C₂₋₆ alkenyl or C₂₋₆ alkynyl, and
L1 is selected from C₁₋₆ alkylene, C₂₋₆ alkenylene or C₂₋₆ alkynylene.

2. The use according to claim 1, wherein R3 is C₂₋₆ alkenyl, R4 is -CN, X1 is O, X2 is O, Q1 is NH, Q2 is NH, Q3 is NH, Q4 is N-R5, R5 is C₁₋₆ alkyl, and L1 is C₁₋₆ alkylene.

3. The use according to claim 1, wherein the compound of Formula (I) is compound CS 12192:

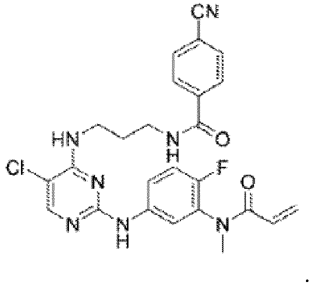

4. The use according to claim 1, wherein the GVHD is an acute GVHD (aGVHD) or a chronic GVHD (cGVHD).

5. The use according to claim 1, wherein the drug is used to improve a survival rate of a patient with GVHD.

6. The use according to claim 1, wherein the drug is used to improve a weight gain of the patient with GVHD.

7. The use according to claim 1, wherein the drug further comprises another active ingredient for preventing or treating GVHD.

8. The use according to claim 7, wherein the another active ingredient is selected from glucocorticoid, calcineurin inhibitor, mycophenolate mofetil, sirolimus, pentostatin, anti-CD25 monoclonal antibody, anti-TNFa monoclonal antibody, ciclosporin, methotrexate, thalidomide, or rapamycin.

9. The use according to claim 8, wherein the glucocorticoid is selected from prednisone or methylprednisolone.
